# EUROPEAN PATENT SPECIFICATION

(11) **EP 1 847 279 B1**
(45) Date of publication and mention of the grant of the patent: **28.01.2009**
(21) Application number: 07014759.0
(22) Date of filing: 05.10.2001
(51) Int. Cl.: A61L 31/10, A61L 31/14, A61L 31/16

(54) **Drug delivery via conformal film**
Arzneimittelfreisetzung über gleichförmigen Film
Administration de médicaments via un film conforme

(43) Date of publication of application: 24.10.2007
(62) Divisional of application: 01308517.0
(73) Proprietor: LARSON, MARIAN L., Bellingham WA 98229-8929 (US); LARSON, EUGENE A., Lummi Island WA 98262 (US)
(72) Inventor: LARSON, MARIAN L., Bellingham WA 98229-8929 (US); LARSON, EUGENE A., Lummi Island WA 98262 (US)
(74) Representative: Gill, Stephen Charles

(56) References cited:
- EP-A- 0 357 793
- WO-A-97/15245
- US-A- 5 674 192
- US-A- 5 958 443
- US-B1- 6 290 729
- US-B1- 6 309 380

## Description

### Field of the Invention

The present invention relates to the local delivery of drugs in vivo into the cardiovascular system and other body regions. In particular, the present invention is directed to the local delivery of drugs by applying a coating of a bioabsorbable/biodegradable or inert, in vivo biocompatible conformal film, to an implantable suture ring.

### Background of the Invention

Angioplasty is a procedure that involves placing and inflating a balloon catheter in the blood vessel in the area of blockage, which breaks up the accumulated plaque and opens the vessel. While this technique works well in the short term, current literature indicates that 30 to 50% of all angioplasty operations performed will need follow-up treatment within six months. This is due to incomplete plaque removal and the formation of scar tissue as a result of irritation of the blood vessel, known as restenosis. Restenosis results in significant morbidity and mortality and often necessitates further interventions such as repeat angioplasty, coronary bypass, laser surgery or local drug delivery. There has been a focused effort in the health-care industry over the last few years to combat restenosis because repeat angioplasty or surgery is expensive, inconvenient, and potentially life threatening.

Limitations of angioplasty long-term success include abrupt closure (4.4 - 8.3%) and restenosis (chronic reclosure 30 - 50%) of the vessel - both of which are associated with excessive vascular injury.

Intravascular stenting (the placement of a supporting structure within a blood vessel) has demonstrated moderate success in addressing these issues. These devices provide structural support to keep the vessel walls from closing and minimize the problem of arterial blockage caused by plaque falling in to the vessel after inflation.

Stents have been made using materials of varied composition. U.S. Patent number 4,886,062 to Wiktor describes a stent made from low memory metal such as a copper alloy, titanium, or gold. Current stent designs tend to be thrombogenic (causing clot formation) and immunologically stimulating (causing cell formation). Current metal stent designs will not eliminate the restenosis problem. If restenosis should recur, follow-up treatments such as laser surgery or localized drug delivery using other angioplasty devices may be required. A stent alone can not restrict hyperplasia of smooth muscle cells, nor can it prevent restenosis or thrombus. Local delivery of antithrombogenic drugs and those capable of restricting hyperplasia of smooth muscle cells is desirable.

Drugs have been incorporated on or in a catheter or stent during the manufacturing design to provide local delivery of drugs to address restenosis, thrombus, and coagulation. U.S. Patents Nos. 4,994,033 to Shockey et al.; 5,674,192 to Sahatjian et al. and 5,545,208 to Wolff et al. disclose catheters comprising absorbable/biodegradable polymers or hydrogels containing the desired dosage of a drug. Stents incorporating drug delivery may be found, for example, in U.S. Patents Nos. 5,766,710 to Turnlund et al.; 5,769,883 to Buscemi et al.; 5,605,696 to Eury et al.; 5,500,013 to Buscemi et al.; 5,551,954 to Buscemi et al. and 5,443,458 to Eury.

When drugs or biological modifiers are applied in conjunction with the manufacture of the device, there are several problems, for example:
1. sterilization: heat or ionizing radiation alters the composition of many drugs and biological modifiers;
2. the presence of a drug imposes a shorter shelf life independent of the implantable medical device, and could require special storage (i.e. refrigeration);
3. the drug dosage is not variable for specific patient needs; and,
4. a large inventory of devices is required to provide a range of drugs and therapies.

It is an object of the present invention to provide a drug delivery system that overcomes the deficiencies associated with the application of drugs in conjunction with the manufacture of the device.

It is a further object of the present invention to provide a procedure where the drug is applied to the device at the point of use of the device.

US 5674192 discloses a catheter with an expandable portion coated with a hydrogel polymer incorporating a drug. EP A 0357793 discloses a suspended release preparation prepared by using alginic acid. US 5958443 discloses gels for drug delivery primarily to the eye or as corneal shield or mask US 6290729 discloses a synthetic barrier which may incorporate bioactive materials and which may be applied to tissue contacting surfaces of implantable medical devices. WO 97/15245 discloses a suturing ring having an impermeable or semi-impermeable coating to prevent tissue ingrowth.

### Summary of the Invention

The present invention is directed to a method of producing an implantable drug-deliverable suture ring according to claim 1.

The present invention is also directed to an implantable drug-deliverable suture ring according to claim 15. For example, the suture ring of a mechanical artificial heart valve is coated with this biodegradable, bio-erodable or bio-inert material containing a drug, cross-linked or cured, or otherwise treated to form a film, immediately prior to placement in the body. When the film-coated device is introduced into the body, the drug contained in the film coating is released in a local region. The invention provides a point of use in vivo drug delivery system whereby the drug and its concentration can be selected by medical personnel immediately prior to implantation of the suture ring.

### Detailed Description of the Preferred Embodiments

The present invention will now be discussed in detail with reference to the preferred embodiments. Unless otherwise stated, all percentages represent weight percent.

Drugs or other biologically active materials incorporated into the drug delivery conformal film system of the present invention are intended to perform a variety of functions, including, but not limited to: anti-clotting or anti-platelet formation, and the prevention of smooth muscle cell growth on the vessel wall. Drugs anticipated for delivery include antibiotics, anticoagulants, tissue generation factor, and angiogenesis drugs. Drugs also include, anti-thrombogenic drugs (heparin, PPACK, enoxaprin, aspirin, coumadin, hirudin. TPA, urokinase, and streptokinase), anti-proliferative drugs (monoclonal antibodies, heparin, angiopeptin, enxoaprin, methotrexate, cisplatin, flourouracil, Adriamycin), antimetabolites, thromboxane inhibitors, non-steroidal and steroidal anti-inflammatory drugs, Beta and Calcium channel blockers, genetic materials (including DNA and RNA fragments), and bioactive materials (such as fibronectin, laminin, elastin, collagen, and intergrins).

The hydrogel is, for example, a thermal irreversible hydrogel.

The invention can be achieved using hydrogels such as thermal irreversible hydrogels that are in vivo biocompatible and biodegradable. One example is a PEO/PEG polymer (e.g. Pluronic manufactured by BASF) combined with an alginate, such as sodium alginate, mixed with water to form a solution and cross-linked by interaction with calcium ions (e.g. immersion in 10% by weight calcium chloride). Hydrogel containing films may exhibit less elasticity than other cross-linked sodium alginate films. However, such hydrogel films are well suited as coatings for devices that are static or unexpanding, such as suture rings

Although the preferred cross-linking agent is calcium chloride, other soluble- substances may be utilized. For example, calcium compounds, such as CaSO₄, magnesium compounds such as MgCl or MgSO₄, or barium compounds are also contemplated by the present invention for use in cross-linking.

While the invention has been described with reference to preferred embodiments it is to be understood that the invention is not limited to the particulars thereof.

## Claims

1. A method of producing an implantable drug-deliverable suture ring, said method comprising:
providing an implantable suture ring,
coating said suture ring with an in vivo biocompatible and biodegradable or bioabsorbable or bioerodable liquid or gel solution containing a polymer, said solution comprising an amount of one or more predetermined drugs, and
converting said liquid or gel solution to a film adhering to said suture ring thereby forming said implantable drug-deliverable medical device,
wherein said suture ring is unexpanding and said film contains a hydrogel.

2. The method of claim 1 wherein said polymer comprises sodium alginate and said solution comprises water and said sodium alginate.

3. The method of claim 2 wherein said solution comprises sodium alginate in an amount of 1 % by weight to 8% by weight.

4. The method of claim 3 wherein said solution comprises sodium alginate in an amount of 4 % by weight.

5. The method of claim 1 wherein the steps of coating and converting are carried out at a point of use of said implantable medical device.

6. The method of claim 1 wherein said converting comprises cross-linking said solution to form said film.

7. The method of claim 6 wherein said cross-linking is effected with a calcium chloride solution.

8. The method of claim 7 wherein said calcium chloride solution comprises about 10% by weight calcium chloride.

9. The method of claim 3 wherein said sodium alginate comprises a molecular weight of 12,000 to 190,000.

10. The method of claim 9 wherein said sodium alginate comprises a molecular weight of 120,000 to 190,000.

11. The method of claim 3 wherein said sodium alginate comprises mannuronic acid in an amount by weight of 58% to 62% and guluronic acid in an amount by weight of 42% to 38%.

12. The method of claim 1 wherein said polymer comprises polyethylene oxide, polyethylene glycol and an alginate.

13. The method of claim 1 wherein said polymer comprises polyethylene glycol.

14. The method of claim 2 wherein said one or more drugs are non-water soluble and wherein up to about 10% of said water is replaced with propylene glycol.

15. An implantable drug-deliverable suture ring, said suture ring comprising:
an implantable medical suture ring, and
a film adhered to said suture ring, said film comprising an in vivo biocompatible and biodegradable or bioabsorbable or bioerodable polymer, said film further comprising an amount of one or more predetermined drugs,
wherein said suture ring is unexpanding and said film contains a hydrogel.

16. The suture ring of claim 15 wherein said polymer comprises sodium alginate.

17. The suture ring of claim 15 wherein said polymer comprises polyethylene glycol or a mixture of polyethylene oxide and polyethylene glycol.

## Patentansprüche

1. Verfahren zur Herstellung eines implantierbaren Nahtrings zur Arzneimittelabgabe, wobei das Verfahren Folgendes umfasst:
die Bereitstellung eines implantierbaren Nahtrings;
das Beschichten des Nahtrings mit einer flüssigen oder gelförmigen Lösung, die in vivo bioverträglich und biologisch abbaubar oder bioabsorbierbar oder bioerodierbar ist und ein Polymer enthält, wobei die Lösung eine Menge eines oder mehrerer vorbestimmter Arzneimittel umfasst; und
das Umwandeln der flüssigen oder gelförmigen Lösung in einen Film, der an dem Nahtring haftet, wodurch die implantierbare medizinische Vorrichtung zur Arzneimittelabgabe gebildet wird,
wobei der Nahtring sich nicht ausdehnt und der Film ein Hydrogel enthält.

2. Verfahren nach Anspruch 1, worin das Polymer Natriumalginat umfasst und die Lösung Wasser und das Natriumalginat umfasst.

3. Verfahren nach Anspruch 2, worin die Lösung Natriumalginat in einer Menge von 1 bis 8 Gew.-% umfasst.

4. Verfahren nach Anspruch 3, worin die Lösung Natriumalginat in einer Menge von 4 Gew.-% umfasst.

5. Verfahren nach Anspruch 1, worin die Schritte des Beschichtens und Umwandelns während der Verwendung der implantierbaren medizinischen Vorrichtung durchgeführt werden.

6. Verfahren nach Anspruch 1, worin das Umwandeln das Vernetzen der Lösung zur Ausbildung des Films umfasst.

7. Verfahren nach Anspruch 6, worin das Vernetzen mit einer Calciumchlorid-Lösung herbeigeführt wird.

8. Verfahren nach Anspruch 7, worin die Calciumchlorid-Lösung etwa 10 Gew.-% Calciumchlorid umfasst.

9. Verfahren nach Anspruch 3, worin das Natriumalginat ein Molekulargewicht von 12.000 bis 190.000 aufweist.

10. Verfahren nach Anspruch 9, worin das Natriumalginat ein Molekulargewicht von 120.000 bis 190.000 aufweist.

11. Verfahren nach Anspruch 3, worin das Natriumalginat Mannuronsäure in einer Menge von 58 bis 62 Gew.-% und Guluronsäure in einer Menge von 42 bis 38 Gew.-% umfasst.

12. Verfahren nach Anspruch 1, worin das Polymer Polyethylenoxid, Polyethylenglykol und ein Alginat umfasst.

13. Verfahren nach Anspruch 1, worin das Polymer Polyethylenglykol umfasst.

14. Verfahren nach Anspruch 2, worin das eine oder die mehreren Arzneimittel nicht wasserlöslich ist/sind und worin bis zu 10 % des Wassers durch Propylenglykol ersetzt werden.

15. Implantierbarer Nahtring zur Arzneimittelabgabe, wobei der Nahtring Folgendes umfasst:
einen implantierbaren medizinischen Nahtring und
einen Film, der an dem Nahtring haftet, wobei der Film ein in vivo bioverträgliches und biologisch abbaubares oder bioabsorbierbares oder bioerodierbares Polymer umfasst und weiters eine Menge eines oder mehrerer vorbestimmter Arzneimittel umfasst,
wobei der Nahtring sich nicht ausdehnt und der Film ein Hydrogel enthält.

16. Nahtring nach Anspruch 15, worin das Polymer Natriumalginat umfasst.

17. Nahtring nach Anspruch 15, worin das Polymer Polyethylenglykol oder ein Gemisch aus Polyethylenoxid und Polyethylenglykol umfasst.

## Revendications

1. Procédé de fabrication d'un anneau de suture implantable distributeur de médicaments, ledit procédé comprenant:
la réalisation d'un anneau de suture implantable,
le revêtement dudit anneau de suture avec une solution liquide ou de gel in vivo biocompatible et biodégradable ou bioabsorbable ou bioérodable contenant un polymère, ladite solution comprenant une quantité d'un ou de plusieurs médicaments prédéterminés, et
la transformation de ladite solution liquide ou de gel en un film adhérent audit anneau de suture en formant ainsi ledit dispositif médical implantable distributeur de médicaments,
où ledit anneau de suture ne se dilate pas, et ledit film contient un hydrogel.

2. Procédé selon la revendication 1, où ledit polymère comprend de l'alginate de sodium, et ladite solution comprend de l'eau et ledit alginate de sodium.

3. Procédé selon la revendication 2, où ladite solution comprend de l'alginate de sodium en une quantité de 1% en poids à 8% en poids.

4. Procédé selon la revendication 3, où ladite solution comprend de l'alginate de sodium en une quantité de 4% en poids.

5. Procédé selon la revendication 1, où les étapes de revêtement et de transformation sont exécutées à un point d'utilisation dudit dispositif médical implantable.

6. Procédé selon la revendication 1, où ladite transformation comprend la réticulation de ladite solution pour former ledit film.

7. Procédé selon la revendication 6, où ladite réticulation est effectuée avec une solution de chlorure de calcium.

8. Procédé selon la revendication 7, où ladite solution de chlorure de calcium comprend environ 10% en poids de chlorure de calcium.

9. Procédé selon la revendication 3, où ledit alginate de sodium comprend un poids moléculaire de 12000 à 190000.

10. Procédé selon la revendication 9, où ledit alginate de sodium comprend un poids moléculaire de 120000 à 190000.

11. Procédé selon la revendication 3, où ledit alginate de sodium comprend de l'acide mannuronique en une quantité en poids de 58% à 62% et de l'acide gluronique en une quantité en poids de 42% à 38%.

12. Procédé selon la revendication 1, où ledit polymère comprend l'oxyde de polyéthylène, le glycol de polyéthylène et un alginate.

13. Procédé selon la revendication 1, où ledit polymère comprend du polyéthylène glycol.

14. Procédé selon la revendication 2, où un ou plusieurs médicaments précités ne sont pas solubles dans l'eau et où jusqu'à environ 10% de ladite eau est remplacé par du propylène glycol.

15. Anneau de suture implantable distributeur de médicaments, ledit anneau de suture comprenant:
un anneau de suture médical implantable, et
un film adhérant audit anneau de suture, ledit film comprenant un polymère in vivo biocompatible et biodégradable ou bioabsorbable ou bioérodable, ledit film comprenant en outre une quantité d'un ou de plusieurs médicaments prédéterminés,
où ledit anneau de suture ne se dilate pas et ledit film contient un hydrogel.

16. Anneau de suture selon la revendication 15, où ledit polymère comprend de l'alginate de sodium.

17. Anneau de suture selon la revendication 15, où ledit polymère comprend du polyéthylène glycol ou un mélange de polyéthylène oxyde et de polyéthylène glycol.
